# EUROPEAN PATENT SPECIFICATION

(11) **EP 1 227 774 B1**
(45) Date of publication and mention of the grant of the patent: **03.09.2003**
(21) Application number: 00973097.9
(22) Date of filing: 08.11.2000
(51) Int. Cl.: A61F 5/01

(54) **ANKLE/FOOT ORTHOSIS**
SPRUNGGELENK-/FUSS- ORTHESE
ORTHESE PEDI-JAMBIERE

(30) Priority: 11.11.1999 GB 9926599
(43) Date of publication of application: 07.08.2002
(73) Proprietor: Tyco Healthcare UK Limited, Gosport, Hampshire PO13 0AS (GB)
(72) Inventor: NICKSON, Shane Willard, Carterknowle Sheffield S7 2EL (GB)
(74) Representative: Chettle, Adrian John
(86) International application number: GB0004288
(87) International publication number: WO01034071

(56) References cited:
- WO-A-98/34572
- US-A- 5 219 324
- US-A- 5 759 168

## Description

This invention relates to an ankle foot orthosis intended to support the lower leg, ankle and foot. Such an orthosis may be required in order to counteract the effects of muscular and nervous disorders.

Conventional orthoses provide a rather rigid strut or frame which is fixed to the lower leg and foot in order to counter the effect of e.g. muscle spasms therein. When fitted, the patient may be able to walk whereas without an orthosis, walking may be almost impossible.

A traditional orthosis is somewhat bulky, and may require the wearing of special clothing and shoes. An additional and significant disadvantage is that a normal walking gait cannot be obtained due to the inevitable immobilisation of at least part of the ankle joint.

WO-A-9834572 proposes an ankle/foot orthosis having a rigid strut connecting a lower leg support to a sole plate. The strut is generally inflexible, but the sole plate is said to have sufficient flexibility to improve the walking gait by permitting bending of the toes.

According to the present invention there is provided an ankle/foot orthosis comprising a strapping member for a lower leg, and having an inflexible anterior longitudinal stiffener, a sole plate, and a relatively flexible strut connecting the longitudinal stiffener and the sole plate, the strut extending to the sole plate at the outer side thereof, and anterior of the position of the ankle.

Such an orthosis is flexible in the region of the ankle, and thus permits a degree of ankle flexibility, which promotes a more normal walking gait.

In this specification the terms flexible and inflexible are relative, and will be understood by the skilled man in relation to ankle/foot orthoses. Generally speaking, a flexible member has the degree of flexibility imparted by glass reinforced plastic resin (grp), whereas an inflexible member has the degree of flexibility imparted by a carbon fibre reinforced plastic resin. In terms of ankle movement, an inflexible member would prevent any substantial ankle movement in the plane or planes intended to be rigidified, whereas a flexible member would permit a degree of movement.

Preferably the flexible portions of the orthosis are manufactured substantially of glass fibre or aramid fibre reinforced resin, and the rigid portion is manufactured in carbon fibre reinforced resin. Such a construction permits integration of the fibres during the lay-up stage of manufacture, thus giving a substantially seamless resilient product after curing.

The rigidity of sole plate is preferably variable over the area thereof, portions being relatively rigid, and portions being relatively flexible so as to give support to the foot whilst allowing bending of the toes.

The strapping member preferably extends around the sides of the leg and has securing straps to accommodate variation in leg thickness. Preferably this strapping member is moulded from a soft thermoplastic so as to be mouldable by application of heat. Thermoplastic also facilitates easy trimming to fit a patient, for example by scissors.

The flexible strut is preferably encased for protection against impact damage, and to reduce abrasion. The encasement may be a sleeve of plastics material, which is preferably heat shrinkable so as to mould itself tightly to the contours of the strut.

Fasteners may be provided whereby the stiffener is movable longitudinally of the strapping member, so as to facilitate comfortable fitment to persons of different leg length.

The stiffener is preferably on the inside of the strapping member, and in a preferred embodiment resides in a channel formed therein. The channel is preferably open at the bottom.

Other features of the invention will be apparent from the following description of a preferred embodiment, shown by way of example only in the accompany drawings in which:

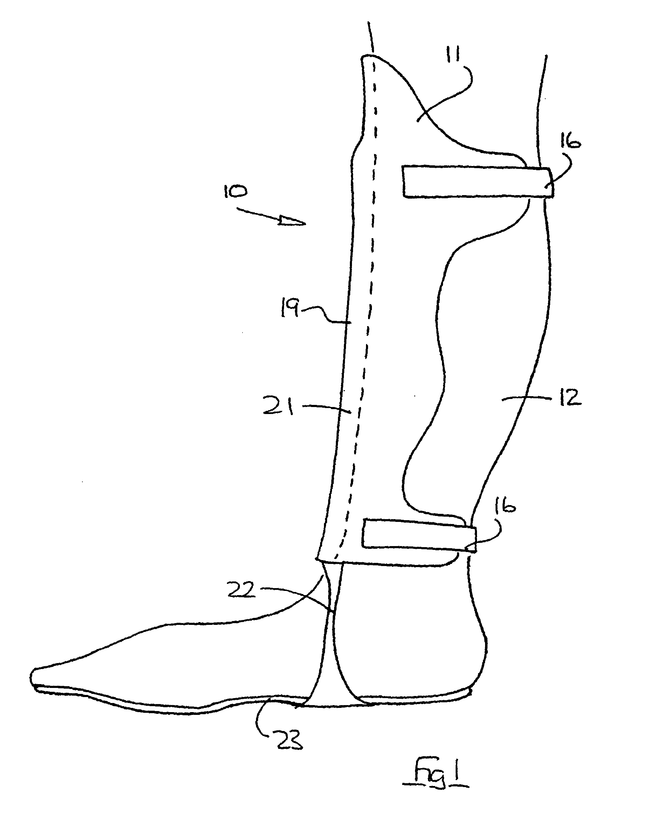

Figure 1 is a side elevation of the lower leg and foot of a human, to which an orthosis according to the invention has been attached;
Figure 2 is a front elevation of an unattached orthosis corresponding to Figure 1; and
Figure 3 is a graphical representation showing the gait provided by the invention.

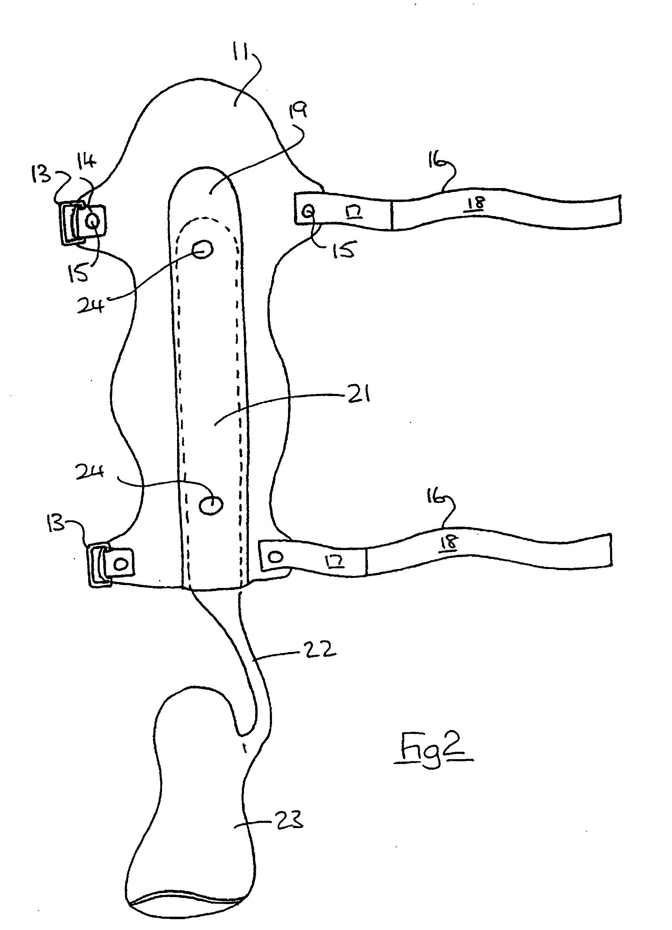

With reference to the drawings, an orthosis 10 comprises a strapping member 11 of thermoplastic and typically having a thickness of 2.5mm. This member is sufficiently rigid to be self-supporting in a shape conforming approximately to the front and side contours of a human leg 12. The member is also mouldable by the application of heat, for example from a hot air gun, in order to obtain a good fit around a patient's leg. The sides of the supporting member are cut away to improve comfort, leaving four strapping regions which extend further round the leg. On the left side of Figure 2 (as viewed) simple buckles 13 are attached by fabric loops 14 and rivets 15. On the right side, fabric straps 16 having inner end and outer end areas 17, 18 covered with the different components of a suitable hook and loop type of fastener. The straps 16 are also attached by rivets 15.

Moulded within the member 11 is a channel 19 open at the bottom and generally aligned with the shin bone.

Closely fitting within the channel 19 is a stiffener 21 of carbon fibre reinforced resin. Typically the stiffener has a width of about 26mm, and a thickness of 3mm.

From the base of the stiffener 21 extends a strut 22 to which is provided a sole plate 23. Both the strut and the sole plate are of glass fibre reinforced resin, but the sole plate may include aramid or carbon fibres in order to improve the toughness and rigidity *of* selected areas. In particular the mid-region of the sole plate may be rather inflexible whereas the toe and heel regions may be relatively flexible so as to improve the gait *of* a wearer. Flexibility of the sole plate may be varied by changing the direction of reinforcement fibres. The periphery of the sole plate may be solely of glass fibre to permit relatively easy trimming to size thereof.

The strut 22 is about 3mm thick at the ends, and has a circular section of about 8mm diameter in the mid-region. The change of strut section is smooth and the ends of the strut are smoothly radiussed into the stiffener and sole plate. During lay-up of the orthosis the fibres are integrated and overlapped to provide an essentially seamless construction.

The sole plate can be of *any* suitable shape, and typically has a thickness of 0.3-2.0mm.

The stiffener 21 is fixed to the strapping member 11 by rivets or screws 24. A plurality of fixing positions may be provided to allow adjustment for length of leg, for example a slot may be provided in the strapping member to allow for adjustment prior to permanent attachment.

Manufacture of the orthosis is by moulding the stiffener, strut and sole plate as a unit, the fibres being laid up in a mould or cut from pre-preg sheet After curing, this component is trimmed to size if necessary. Trimming may be cosmetic, or can be used to influence the degree of flexibility imparted by the strut 22.

Thereafter the strapping portion is attached, after suitable measurement of a patient Final fitting includes trimming of the sole plate, and moulding or trimming of the strapping portion.

To attach the orthosis, straps 16 are passed through the buckles 13, and the portions 18 laid over the portions 17 to the required degree of tightness. The straps 16 may finally be trimmed to a reasonable length.

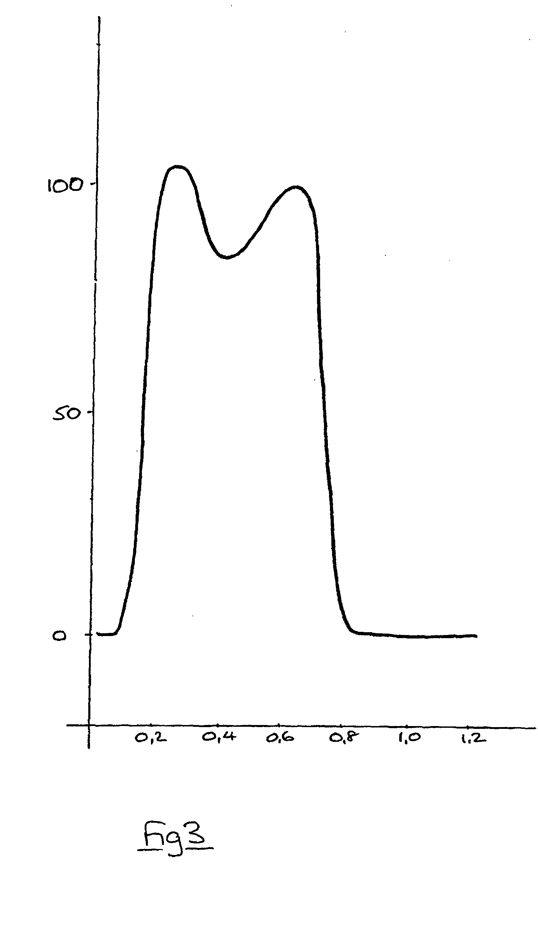

Figure 3 shows a graphical representation of the gait of a typical wearer of the preferred embodiment. The 'X' axis shows time in seconds, and the 'Y' axis shows percentage of body weight. This gait approximates closely to a normal gait, the first peak indicating a distinct heel strike, and the second peak indicating a distinct toe strike. The forces generated approach those of a normal gait thus giving the user the appearance of walking normally. The overall time for a step at normal walking pace is about the same as for an undisabled person, thus permitting an approximately even walking pace where only one leg of the person requires support.

Various modification to the embodiment will occur to the skilled man within the scope of the appended claims.

## Claims

1. An ankle/foot orthosis comprising a strapping member (11) for a lower leg, and having an inflexible anterior longitudinal stiffener (21), a sole plate (23), and a strut (22) connecting the longitudinal stiffener (21) and the sole plate (23), the strut (22) extending to the sole plate (23) at the outer side thereof, and anterior of the position of the ankle, **characterized in that** said strut (22) is flexible.

2. An ankle/foot orthosis according to claim 1 wherein the flexible portions of the orthosis are manufactured substantially of glass fibre or aramid fibre reinforced resin, and the inflexible portion is manufactured in carbon fibre reinforced resin.

3. An ankle/foot orthosis according to claim 1 or claim 2 wherein the rigidity of sole plate (23) varies over the area thereof, portions being inflexible, and portions being flexible so as to give support to the foot whilst allowing bending of the toes.

4. An ankle/foot orthosis according to any preceding claim wherein the strapping member (11) is adapted to extend around the sides of a leg, and has securing straps adapted to accommodate variation in leg thickness.

5. An ankle/foot orthosis according to claim 4 wherein said strapping member (11) is moulded from a soft thermoplastic so as to be mouldable by application of heat

6. An ankle/foot orthosis according to any preceding claim wherein said strut (22) is encased.

7. An ankle/foot orthosis according to claim 6 wherein the strut (22) is encased in a sleeve of plastics material.

8. An ankle/foot orthosis according to claim 7 wherein said sleeve is heat shrinkable so as to mould itself tightly to the contours of the strut (22).

9. An ankle/foot orthosis according to any preceding claim wherein fasteners (24) are provided whereby the stiffener (21) is movable longitudinally of the strapping member (11).

10. An ankle/foot orthosis according to claim 9 wherein said stiffener (21) is on the inside of said strapping member (11).

11. An ankle/foot orthosis according to claim 10 wherein said stiffener (21) resides in a channel (19) of the strapping member (11).

12. An ankle/foot orthosis according to claim 11 wherein said channel (19) is open at the bottom.

## Patentansprüche

1. Fußgelenk/Fuß-Orthese mit einer Bügelverbindungseinrichtung (11) für den unteren Fußbereich und einem unflexiblen vorderen Längsversteifungsteil (21), einer Sohlenplatte (23) und einer Strebe (22), welche das Längsversteifungsteil (21) und die Sohlenplatte (23) verbindet, wobei die Strebe (22) sich an der Außenseite zu der Sohlenplatte (23) und einer äußeren Position des Fußgelenks erstreckt, **dadurch gekennzeichnet, daß** die Strebe (22) flexibel ist.

2. Fußgelenk/Fuß-Orthese nach Anspruch 1, bei der die flexiblen Abschnitte der Orthese im wesentlichen aus mit Glasfaser- oder Aramidfaser verstärktem Harz hergestellt sind, und der unflexible Abschnitt aus Kohlefaser verstärktem Harz hergestellt ist.

3. Fußgelenk/Fuß-Orthese nach Anspruch 1 oder 2, bei der die Steifigkeit der Sohlenplatte (23) sich über den Erstreckungsbereich verändert, wobei Abschnitte unflexibel und Abschnitte flexibel sind, um dem Fuß einen Halt zu verleihen und zugleich ein Beugen der Zehen zu gestatten.

4. Fußgelenk/Fuß-Orthese nach einem der vorangehenden Ansprüche, bei der die Bügelverbindungseinrichtung (11) derart beschaffen und ausgelegt ist, daß sie sich um die Seiten eines Beines erstreckt, und Befestigungsbänder hat, welche derart beschaffen und ausgelegt sind, daß sie unterschiedliche Beindicken aufnehmen.

5. Fußgelenk/Fuß-Orthese nach Anspruch 4, bei der die Bügelverbindungseinrichtung (11) aus einem weichen, thermoplastischen Material derart ausgeformt ist, daß es mittels Einwirkung von Wärme formbar ist.

6. Fußgelenk/Fuß-Orthese nach einem der vorangehenden Ansprüche, bei der die Strebe (22) umschlossen ist.

7. Fußgelenk/Fuß-Orthese nach Anspruch 6, bei der die Strebe (22) in einer Hülse aus Kunststoffmaterial eingeschlossen ist.

8. Fußgelenk/Fuß-Orthese nach Anspruch 7, bei der die Hülse derart wärmeschrumpfbar ist, daß sie sich selbsttätig eng an die Konturen der Strebe (22) passend anlegt.

9. Fußgelenk/Fuß-Orthese nach einem der vorangehenden Ansprüche, bei der Befestigungsmittel (24) vorgesehen sind, und das Versteifungsteil (23) in Längsrichtung der Bügelverbindungseinrichtung (11) beweglich ist.

10. Fußgelenk/Fuß-Orthese nach Anspruch 9, bei der das Versteifungsteil (21) auf der Seite der Bügelverbindungseinrichtung (11) angeordnet ist.

11. Fußgelenk/Fuß-Orthese nach Anspruch 10, bei der das Versteifungsteil (21) in einem Kanal (19) der Bügelverbindungseinrichtung (11) angeordnet ist.

12. Fußgelenk/Fuß-Orthese nach Anspruch 11, bei der der Kanal (19) am Boden offen ist.

## Revendications

1. Orthèse cheville/ pied comprenant un organe de bandage (11) pour une partie inférieure de jambe, et comportant un raidisseur longitudinal antérieur non flexible (21), une plaque de semelle (23) et un étai (22) reliant le raidisseur longitudinal (21) et la plaque de semelle (23), l'étai (22) s'étendant jusqu'à la plaque de semelle (23) au côté extérieur de celle-ci, et antérieurement à la position de la cheville, **caractérisée en ce que** ledit étai (22) est flexible.

2. Orthèse cheville/ pied selon la revendication 1 dans laquelle les parties flexibles de l'orthèse sont fabriquées essentiellement en fibre de verre ou en résine renforcée en fibre d'aramide, et la partie non flexible est fabriquée en résine renforcée en fibre de carbone.

3. Orthèse cheville/ pied selon la revendication 1 ou 2, dans laquelle la rigidité de la plaque de semelle (23) varie sur la surface de celle-ci, des parties étant non flexibles, et des parties étant flexibles de façon à donner un support au pied tout en permettant une flexion des orteils.

4. Orthèse chevillel/ pied selon l'une quelconque des revendications précédentes, dans laquelle l'organe de bandage (11) est adapté pour s'étendre autour des côtés d'une jambe, et comporte des bandes de fixation adaptées pour absorber une variation d'épaisseur de la jambe.

5. Orthèse cheville/ pied selon la revendication 4 dans laquelle ledit organe de bandage (11) est moulé à partir d'une matière thermoplastique molle, de façon à pouvoir être moulé par application de chaleur.

6. Orthèse cheville/ pied selon l'une quelconque des revendications précédentes, dans laquelle ledit étai (22) est enrobé.

7. Orthèse cheville/ pied selon la revendication 6, dans laquelle l'étai (22) est enrobé dans un manchon de matière plastique.

8. Orthèse cheville/ pied selon la revendication 7 dans laquelle ledit manchon est rétrécissable à la chaleur, de façon à se mouler étroitement aux contours de l'étai (22).

9. Orthése cheville/ pied selon l'une quelconque des revendications précédentes, dans laquelle des éléments de fixation (24) sont agencés, ce par quoi le raidisseur (21) est déplaçable longitudinalement sur l'organe de bandage (11).

10. Orthèse cheville/ pied selon la revendication 9 dans laquelle ledit raidisseur (21) est à l'intérieur de l'organe de bandage (11).

11. Orthèse cheville/ pied selon la revendication 10, dans laquelle ledit raidisseur (21) est situé dans un canal (19) de l'organe de bandage (11).

12. Orthèse cheville/ pied selon la revendication 11 dans laquelle ledit canal (19) est ouvert en partie inférieure.
